**Europäisches Patentamt**

**European Patent Office**

**Office européen des brevets**

(19)

(11) Veröffentlichungsnummer: **0 475 542 A1**

## (12) EUROPÄISCHE PATENTANMELDUNG

(21) Anmeldenummer: **91250198.8**

(22) Anmeldetag: **18.07.91**

(51) Int. Cl.⁵: **C02F 3/10**, C02F 1/28, C12N 11/04, C02F 1/62

(30) Priorität: **24.08.90 DE 4027219**

(43) Veröffentlichungstag der Anmeldung: **18.03.92 Patentblatt 92/12**

(84) Benannte Vertragsstaaten: **DE DK FR GB IT NL**

(71) Anmelder: **Preussag Noell Wassertechnik GmbH**
**Alfred-Nobel-Strasse 20**
**W-8700 Würzburg 1(DE)**

(72) Erfinder: **Wildenauer, Franz Xaver, Dr.**
**Dorfstrasse 1b**
**W-2861 Ritterhude(DE)**
Erfinder: **Menzel, Roman, Dr.**
**Ouellenweg 10**
**W-4450 Lingen(DE)**
Erfinder: **Vetter, Helmut**
**Oslebshauser Heerstrasse 156**
**W-2800 Bremen 21(DE)**
Erfinder: **Vorlop, Klaus-Dieter, Dr.**
**Hochstrasse 7**
**W-3300 Braunschweig(DE)**
Erfinder: **Remmers, Peter**
**Lessingplatz 6**
**W-3300 Braunschweig(DE)**

(74) Vertreter: **Kaiser, Henning**
**Preussag AG, Patente und Lizenzen,**
**Postfach 15 12 27, Kurfürstendamm 32**
**W-1000 Berlin 15(DE)**

(54) **Verfahren und Anlage zur Entfernung von Schwermetallen aus wässrigen Medien durch Bioadsorber.**

(57) Zur Entfernung von Schwermetallen aus wässrigen Medien werden Bioadsorber vorgeschlagen. Diese enthalten in Gelkugeln polymerfixierte Mikroorganismen, die die Fähigkeit besitzen, in geringer Konzentration (z. B. weniger als 10 mg/l) in Wasser enthaltene Schwermetalle selektiv zu adsorbieren. Die Gelkugeln werden aus einer geeignete Mikroorganismen enthaltenden Polyvinylalkohol-Lösung dadurch hergestellt, daß die Lösung in eine tiefkalte Flüssigkeit wie Stickstoff eingetropft wird. Durch sehr langsames Auftauen werden Gelkugeln erhalten, die große Elastizität und Abriebfestigkeit aufweisen und daher auch über längere Zeit in einem Reaktor zirkulieren können. Eine Regenerierung der Bioadsorber ist möglich, und die Schwermetalle können aus der Regenerierlösung ausgefällt werden.

Die Erfindung betrifft ein Verfahren und eine Anlage zur Entfernung von Schwermetallen aus wässrigen Medien durch Bioadsorber. Die Schwermetalle können in Trinkwasser oder Abwasser vorliegen. Schwermetalle werden üblicherweise aus Wasser durch Fällung mittels Kalk oder sulfidischen Verbindungen oder durch Adsorption an Ionenaustauscher entfernt. Die Entfernung gelingt jedoch auf diese Weise nur bis zu einem Restwert von 2 bis 10 mg/l. Eine darüberhinausgehende Beseitigung der Schwermetalle aus Wasser ist bisher nur durch Eindampfen beziehungsweise Destillation mit einem erheblichen Energieaufwand möglich.

Die vorliegende Erfindung nutzt die Eigenschaften bestimmter Mikroorganismen, Schwermetalle noch in sehr geringen Konzentrationen selektiv zu adsorbieren, d. h. anzureichern. Diese Eigenschaft kann sowohl bei lebenden wie toten Mikroorganismen vorhanden sein. Geeignete Mikroorganismen sind in der Literatur bekannt. Die Mikroorganismen mit den adsorbierten Schwermetallen sind wegen ihrer geringen Größe nur mit hohem Aufwand aus Wasser wieder abzutrennen und müssen andererseits in hohen Konzentrationen in das Wasser eingebracht werden.

Es wurde daher versucht, die Mikroorganismen in Alginat-Polymer-Kügelchen zu fixieren um sie konzentrierter einzusetzen und sie aus dem behandelten Wasser besser abscheiden zu können. Gelkugeln aus z. B. Alginat, Carrageenan und ähnlichen Materialien weisen eine zu geringe chemische, mechanische und biologische Stabilität auf, um in größerem Maße eingesetzt zu werden. Andere Polymere, wie beispielsweise auch Polyurethan, besitzen eine geringe Durchlässigkeit und eine geringere innere Porosität, wobei beides die Erhöhung des Diffusionswiderstandes bewirkt. Es ist daher noch keine technisch realisierte Anwendung derartiger Gelkörper bekannt geworden.

Die Erfindung hat sich daher zur Aufgabe gemacht, verbesserte Bioadsorber in Form von Kugeln oder Perlen zu schaffen, die zur Entfernung von in geringer Konzentration in wässrigen Medien vorhandenen Schwermetallen unter wirtschaftlich vertretbaren Bedingungen geeignet sind und eine für die Anwendung der Bioadsorber vorgesehene Anlage vorzuschlagen.

Die Lösung dieser Aufgabe ist in den Ansprüchen 1 und 9 angegeben. Die Unteransprüche beziehen sich auf bevorzugte Weiterbildungen.

Es wurde gefunden, daß aus einer wässrigen Lösung eines Polyvinylalkohols mit einem Polymerisierungsgrad über 1200 und einem Hydrolysegrad von mindestens 98% mit einem Verhältnis von 40 bis 150 g Polyvinylalkohol, vorzugsweise 70 bis 100 g zu 1 l Wasser eine hochviskose Lösung hergestellt werden kann, daß aus dieser Lösung durch Eintropfen in eine tiefkalte Flüssigkeit von unter -30° C, vorzugsweise in flüssigen Stickstoff kugelförmige Körper hergestellt werden können und daß sich aus diesen gefrorenen Körpern bei langsamem Auftauen, insbesondere im Temperaturbereich zwischen -20° C und 5° C mit einer Erwärmungsgeschwindigkeit von nicht mehr als 20° C/h, vorzugsweise bis etwa 4° C/h zwischen -5 und +5° C stabile und elastische Gelkugeln hergestellt werden können, in denen die der Lösung zugegebenen Mikroorganismen als Bioadsorber im Polymer fixiert sind. Der hochviskosen Polyvinylalkohol-Lösung kann eine weichmachende, die Elastizität der hergestellten Gelkugeln verbessernde Komponente, wie Glyzerin, Zucker oder wasserlösliche organische Verbindungen mit Hydroxylgruppen bis zu 40 Gew.-% zugefügt werden.

Die hergestellten Gelkugeln besitzen eine glatte abriebfeste Oberfläche und sind elastisch deformierbar. Sie sind jedoch weitgehend abriebfest und werden durch in Reaktionsbehältern austretende Scherkräfte sowie durch in dem zu behandelnden Wasser enthaltenen chemischen Bestandteile nicht zerstört.

In die zur Herstellung der Gelkugeln verwendete Lösung können Hilfsstoffe zugemischt werden, die die Wirkung der Mikroorganismen unterstützen. Derartige Hilfsstoffe sind ebenfalls in der Lage, Schwermetalle zu adsorbieren. Sie können daher als Puffer Belastungsstöße ausgleichen und die Mikroorganismen vor erhöhten Schwermetallkonzentrationen schützen.

Bioadsorber werden zweckmäßigerweise dann eingesetzt, wenn nur noch eine geringe Konzentration von Schwermetallen in dem wässrigen Medium vorhanden ist. Ihre Verwendung kann sinnvoll sein, wenn die Konzentration weniger als 10 mg/l beträgt. Ist ein wässriges Medium höher mit Schwermetallen belastet, so werden zunächst durch Fällungsmittel, wie Kalk oder sulfidische Verbindungen, Schwermetalle als unlösliche Metallverbindungen ausgefällt. Alternativ kann die Entfernung von Metallen auch durch Ionenaustauscher-Materialien erreicht werden. Diese bekannten Methoden sind als Vorstufe für die Behandlung durch Bioadsorber notwendig, werden jedoch unwirtschaftlich, wenn Restkonzentrationen von unter 10 mg/l beseitigt oder wenigstens weiter reduziert werden sollen. Wässrige Medien mit einer geringen Restkonzentration werden daher durch Reaktorbehälter gepumpt, in denen sich die Bioadsorber befinden.

Es können mehrere Reaktoren hintereinander geschaltet werden, in denen sich Bioadsorber befinden, die eine unterschiedliche Affinität zu den verschiedenen Schwermetallen besitzen, so daß in jedem Reaktor selektiv ein anderes Metall in dem jeweiligen Bioadsorber akkumuliert wird. Es ist auch möglich Bioadsorber mit unterschiedlichen Mikroorganismen in einem Reaktor zu verwenden, so daß gleichzeitig verschiedene Schwermetalle durch diejenigen Bioadsorber aufgenommen werden, die eine entsprechende Affinität zu den Metallen besitzen.

Die Bioadsorber, in denen sich über längere Zeiträume zunehmend Schwermetalle anreichern, können durch eine Regenerierlösung desorbiert werden. Zur Regenerierung können sie kurzzeitig, z. B. mit verdünnter Säure oder mit einer Lösung von Komplexbildnern gespült werden, die aus den Kugeln die Schwermetalle herauslöst. Die in die Regenerierlösung aufgenommenen Schwermetalle werden aus dieser gegebenenfalls nach Anhebung des pH-Wertes mit Hilfe von Kalk oder sulfidischen Verbindungen ausgefällt.

Zweckmäßigerweise werden die Bioadsorber in bestimmten Zeitabständen, beispielsweise nach einer Regenerierung wieder stärker aktiviert, indem eine Nährlösung zugeführt wird und Bedingungen eingestellt werden, die die Mikroorganismen zum Wachsen anregt. Hierdurch werden etwa durch die Schwermetalle oder die Regenerierlösung beeinträchtigte Mikroorganismen wieder auf ihre anfängliche Fähigkeit zum Adsorbieren zurückgebracht, so daß das Verfahren mit denselben Bioadsorbern auch über einen längeren Zeitraum durchgeführt werden kann.

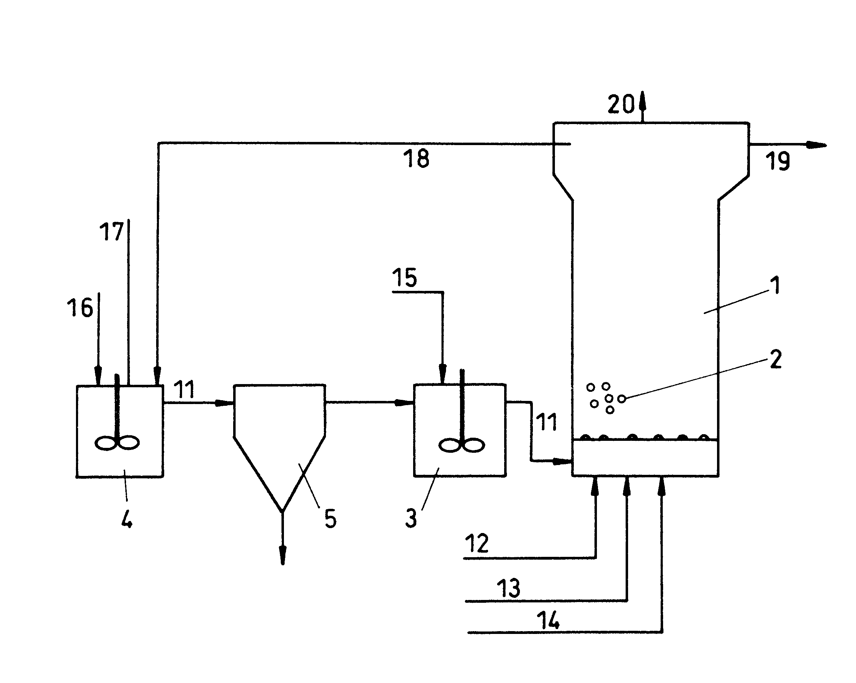

Die beigefügte Zeichnung zeigt schematisch eine Anlage zur Entfernung von Schwermetallen mittels Bioadsorber.

In einem Reaktor 1 befinden sich die Bioadsorber 2 in Form von Gelkugeln oberhalb eines durchlässigen Bodens. In den unteren Teil des Reaktors führt eine Leitung 11 für das wässrige Medium, eine Leitung 12 für ein Nährmedium, eine Leitung 13 für eine Regenerierflüssigkeit und eine Leitung 14 für Luft oder Sauerstoff. Die Leitungen sind in nicht dargestellter Weise absperrbar, so daß beispielsweise Regenerierflüssigkeit oder Nährmedium nur zu gewissen Zeiten zugeführt werden, während kein weiteres wässriges Medium zugeleitet wird. Das behandelte Medium fließt über eine Leitung 19 ab, die gegebenenfalls auch zu einem zweiten Reaktor führen kann, in dem andere Bioadsorber ein anderes Schwermetall akkumulieren. Das wässrige Medium wird zweckmäßigerweise durch einen Fällungsvorgang auf eine niedrige Konzentration vorbehandelt. Hierzu dient ein Fällungsbehälter 4, dem das kontaminierte Abwasser über eine Leitung 16 und das Fällungsmittel über eine Zuführung 17 zugeführt werden. Der Behälter weist eine Einrichtung zur Durchmischung der Lösung auf. Die Lösung wird einem Abscheider 5 zugeführt, in der sich der gefällte Schwermetallschlamm absetzt. Das Medium mit dem geringen Restgehalt gelangt in einen Behälter 3, wo durch Zugabe von Lauge durch Leitung 15 der pH-Wert eingestellt werden kann. Aus dem Behälter 3 fließt das Medium über Leitung 11 in den Reaktor 1. Während der Regenerierung wird die Regenerierflüssigkeit, in der sich aus den Bioadsorbern herausgelöste Schwermetalle anreichern, getrennt abgeführt und zweckmäßigerweise dem Fällungsbehälter zugeleitet. An dem Reaktor 1 ist weiterhin eine Leitung 20 zur Abführung der Abluft vorgesehen, durch die überschüssige Luft und gegebenenfalls weitere Gase aus dem wässrigen Medium austreten können. Durch die erfindungsgemäße Verwendung von Gelkörpern mit Bioadsorbern aus Polyvinylalkohol läßt sich erstmals ein in einem technischen Verfahren einsetzbarer Bioadsorber herstellen. Das Verfahren ermöglicht darüberhinaus erstmals die Regeneration der adsorbierenden Aktivität durch nachträgliche Anzucht der Mikroorganismen, wenigstens aus in den Gelkörpern verbliebenen Resten als Teil des Prozesses. Dadurch können auch verhältnismäßig teuer zu produzierende Mikroorganismen bei dem erfindungsgemäßen Verfahren verwendet werden.

## Patentansprüche

1. Verfahren zur Entfernung von Schwermetallen aus wässrigen Medien durch Bioadsorber in Reaktoren mittels in Gelkugeln polymerfixierter Mikroorganismen, wobei die Gelkugeln aus einer wässrigen Lösung eines Polyvinylalkohols mit einem Hydrolysegrad von mindestens 98% und einem Verhältnis von 40 bis 150 g Polyvinylalkohol zu einem Liter Wasser durch Eintropfen dieser Lösung in eine wenigstens unter -30° C kalte Flüssigkeit und anschließendes Auftauen der gefrorenen Kugeln im Temperaturbereich von -20° C bis 5° C mit einer Erwärmungsrate von bis zu etwa 20° C/h hergestellt werden und wobei der wässrigen Lösung lebende oder tote Spezies von Mikroorganismen, wie Bakterien, Hefen oder Pilze zugesetzt werden, die Schwermetalle selektiv adsorbieren.

2. Verfahren nach Anspruch 1, dadurch gekennzeichnet, daß der Lösung zur Herstellung der Gelkugeln Hilfsstoffe zugemischt werden, die die Wirkung der Mikroorganismen unterstützen, indem sie ebenfalls wenigstens zeitweilig Metalle adsorbieren und daher als Puffer Belastungsstöße ausgleichen.

3. Verfahren nach Anspruch 1 oder 2, dadurch gekennzeichnet, daß vor der Behandlung mit den Bioadsorbern die Konzentration der Schwermetalle in dem wässrigen Medium auf weniger als 10 mg/l durch Ausfällung als in Wasser unlösliche Metallverbindungen oder

Adsorbtion durch Ionenaustauscher-Materialien verinegert wird.

4. Verfahren nach einem oder mehreren der vorangehenden Ansprüche, dadurch gekennzeichnet, daß die Entfernung unterschiedlicher Schwermetalle in mehreren aufeinander folgenden Reaktoren erfolgt, in denen sich Bioadsorber befinden, die eine unterschiedliche Affinität zu den verschiedenen Schwermetallen besitzen, so daß in jedem Reaktor ein anderes Metall akkumuliert wird.

5. Verfahren nach einem der Ansprüche 1 bis 3, dadurch gekennzeichnet, daß in einem Reaktor verschiedene Gelkugeln mit je einer Spezies von Mikroorganismen, die eine Affinität zu einem Schwermetall aufweist, untergebracht sind.

6. Verfahren nach einem oder mehreren der vorangehenden Ansprüche, dadurch gekennzeichnet, daß die Schwermetalle aus dem Bioadsorber durch eine Regenerierlösung desorbiert werden, die in Form einer verdünnten Säure oder einer Lösung von Komplexbildnern kurzfristig durch den Reaktor geleitet wird.

7. Verfahren nach Anspruch 6, dadurch gekennzeichnet, daß die in der Regenerierlösung angereicherten Schwermetalle gegebenenfalls nach Anhebung des pH-Wertes mit Hilfe von Kalk oder sulfidischen Verbindungen ausgefällt werden.

8. Verfahren nach einem oder mehreren der vorangehenden Ansprüche, dadurch gekennzeichnet, daß die Bioadsorber, insbesondere nach der Desorbierung durch die Regenerierlösung dadurch aktiviert werden, daß sie mit einer Nährlösung behandelt und Bedingungen eingestellt werden, die die Mikroorganismen zum Wachstum anregen.

9. Anlage zur Durchführung des Verfahrens nach einem oder mehreren der Ansprüche 1 bis 8, dadurch gekennzeichnet, daß die Anlage umfaßt

a) wenigstens einen Reaktor (1), der die Bioadsorber (2) enthält und mit Anschlüssen für das wässrige Medium (11), für ein Nährmedium (12), für eine Regenerierflüssigkeit (13) und für Luft oder Sauerstoff (14) mit Einrichtungen zur Verteilung dieser Gase in dem Reaktor,

b) einem dem Reaktor (1) vorgeschalteten Behälter (3) mit einer Mischeinrichtung und einer Zuführung für Lauge (15) zur Einstellung des pH-Wertes und weiter einen Fällungsbehälter (4) vorgeschaltet ist, dem das kontaminierte Abwasser über eine Leitung (16) und eine Fällungsmittel über eine Zuführung (17) zugeführt werden und an den sich ein in der Leitung (11) angeordneter Abscheider (5) zum Abtrennen von Schwermetallschlamm nach der Fällung anschließt, einer Leitung (18) von dem Reaktor (1) zu dem Fällungsbehälter (4), um während der Regenerierung die Regenerierflüssigkeit in den Fällungsbehälter zu leiten,

c) einer Leitung (19) zur Abführung des behandelten wässrigen Mediums und

d) einer Abluftleitung (20) der zugeführten aber nicht verbrauchten Gase sowie gegebenenfalls weiterer sich aus dem wässrigen Medium lösender Gase.

4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | ENVIRONMENTAL SCIENCE AND TECHNOLOGY. Bd. 24, Nr. 2, Februar 1990, EASTON, PA US Seiten 220 - 228; PATRICIA O. HARRIS ET AL.: 'BINDING OF METALIONS BY PARTICULATE BIOMASS DERIVED FROM cHLORELLA VULGARIS AND SCENEDESMUS QUADRICAUDA' * Seite 220, linke Spalte, Zeile 1 - Seite 221, linke Spalte, Absatz 4 * * * Seite 221, rechte Spalte, Zeile 36 - Zeile 51 EP 91250198030* * * * Page 227, SUMMARY * * | 1,6 | C 02 F 3/10 C 02 F 1/28 C 12 N 11/04 C 02 F 1/62 |
| | – – – | | |
| A | CHEMIE. INGENIEUR. TECHNIK. Bd. 62, Nr. 7, Juli 1990, WEINHEIM DE Seiten 582 - 583; MARKUS RÖHRICHT ET AL.: 'ABTRENNUNG VON SCHWERMETALLEN AUS AB-WASSERSTRöMEN - BIOSORPTION IM VERGLEICH ZU HERKöMMLICHEN VERFAHREN' * Seite 582, linke Spalte, Absatz 4 - Seite 583, rechte Spalte, Absatz 2 * * | 1,6 | |
| | – – – | | |
| A | FR-A-2 223 078 (HITACHI) * Seite 11; Ansprüche 1,2,5 * * | 1 | |
| | – – – | | |
| A | EP-A-0 320 483 (MONSANTO) * Spalte 11; Ansprüche 1-3,6,10,11 * * * Spalte 2, Zeile 10 - Zeile 23 * * * Spalte 2, Zeile 33 - Zeile 47 * * * Spalte 4, Zeile 30 - Spalte 5, Zeile 15 * * * Spalte 5, Zeile 41 - Zeile 48 * * * Spalte 5, Zeile 58 - Spalte 6, Zeile 16 * * | 1,2 | **RECHERCHIERTE SACHGEBIETE (Int. Cl.5)** C 02 F C 12 N |
| | – – – | | |
| A | PATENT ABSTRACTS OF JAPAN vol. 10, no. 335 (C-384)(2391) 13. November 1986 & JP-A-61 139 385 ( SUSUMU HASHIMOTO ) 26. Juni 1986 * Zusammenfassung * * | 1 | |
| | – – – | | |
| A | WO-A-8 700 161 (EPOC LIMITED) * Seite 17, Zeile 2 - Zeile 25; Abbildung 1 * * | 9 | |
| | – – – – – | | |

Der vorliegende Recherchenbericht wurde für alle Patentansprüche erstellt

| Recherchenort | Abschlußdatum der Recherche | Prüfer |
|---|---|---|
| Den Haag | 15 Januar 92 | TEPLY J. |